# EUROPEAN PATENT SPECIFICATION

(11) **EP 3 283 032 B1**
(45) Date of publication and mention of the grant of the patent: **01.09.2021**
(21) Application number: 16716626.3
(22) Date of filing: 18.04.2016
(51) Int. Cl.: A61F 13/532, A61F 13/15, B29C 65/00, D04H 1/407, D04H 1/413

(54) **EQUIPMENT AND PROCESS FOR CREATING PARTICLE FREE REGIONS IN A PARTICLE LOADED FIBROUS WEB**
VORRICHTUNG UND VERFAHREN ZUR ERZEUGUNG PARTIKELFREIER REGIONEN IN EINER PARTIKELBELADENEN FASERSTOFFBAHN
ÉQUIPEMENT ET PROCÉDÉ PERMETTANT DE CRÉER DES RÉGIONS DÉPOURVUES DE PARTICULES DANS UN VOILE FIBREUX CHARGÉ DE PARTICULES

(30) Priority: 17.04.2015 GB 201506563
(43) Date of publication of application: 21.02.2018
(73) Proprietor: Concepts For Success (C4S), 53881 Euskirchen (DE)
(72) Inventor: SCHMITZ, Christoph, 53881 Euskirchen (DE)
(74) Representative: Plischke, Manfred
(86) International application number: PCT/EP2016/058524
(87) International publication number: WO 2016/166368

(56) References cited:
- WO-A1-2013/163388
- WO-A1-2014/001487
- WO-A1-2014/010365
- US-A1- 2002 039 867

## Description

### Field of the invention

The present invention relates to the handling and an equipment for the handling of a fibrous web that comprises particulate additives, which are positioned within interstices between the fibers and/or on the surface of the webs. By applying the present invention, predetermined regions are created in the web, which are essentially free of the particulate additives.

### Background

For various applications, predetermined particle free regions desired, for example when a material, such as a liquid or a solidifying liquid, is to be added to the web, and which should be separated from the particulate additives. A wide application area is related to the bonding of webs, where the bonding regions should be particle free. Such bonding may be by applying glues or adhesives or by applying melt fusion bonding.

A particular area, where the present invention can be applied, is the manufacturing of absorbent structures for absorbent articles like diapers or the like, where particles, such as superabsorbent polymer particles, are removed from predetermined regions of a fibrous web, such that good bonding can take place in these predetermined regions, such as by meltfusion bonding, such as by introducing ultrasonic energy, or by the addition of an adhesive or glue to these regions, such that the web can be bonded in itself or to other materials, such as other webs.

In a first approach, free flowing particles are "printed" onto a web surface and entangled by fibrous thermoplastic adhesive, such as disclosed in EP1621165 (Blessing et al, P&G). As the web surface is indented, pockets are formed where the deposited particles are confined. Between neighboring pockets the web is supported by pins. The pin supported regions ae essentially particle free.

In a similar approach, such as described in WO2012052173A1 air blowing is used in order to aim at particle fee bonding regions.

It is well known to bond webs by guiding the web through a gap between two compressing tools, often two rolls or profiled belts, at least one of which comprises pins or other protrusions such that predetermined regions of the web are more compressed than other regions, and wherein the bonding takes place in these more compressed regions, also referred to as bond points or bonding regions.

One bonding method often used is roller bonding. A bonding roller may have its outer cylindrical surface etched, machined or otherwise formed to have a pattern of bonding surfaces with recessed, areas between them. It may be mated with a second roller also having a bonding pattern, or alternatively having a smooth, featureless cylindrical surface. The two rollers may be disposed with their axes parallel and. their cylindrical surfaces in contact or near contact, forming a compression passage or gap. When the fiber aggregate or a filament batt is passed between the two rollers, it is compressed and the bonding pattern of the roller is impressed into the batt. Accordingly, in WO2013/16338 a process and the resulting web are described, wherein rolls with inflexible protrusions create a pattern of ridges and grooves in a web material.

In some applications one or both rollers may be heated, or energy may be supplied, (such as ultrasonic energy) that imparts to or generates heat energy in the fibers and/or filaments as the batt passes between the rollers. In a suitably controlled process for consolidating and bonding a web including thermoplastic fibers and/or filaments, the heating energy may be controlled, to cause the material(s) forming fibers and/or filaments superimposed and compressed beneath bonding surfaces on the roller to melt, flow together, and for miscible materials, fuse, creating a pattern of thermal bonds corresponding to the pattern of bonding surfaces on the bonding roller.

A particular execution creating melt-fusion bonding is described in WO2012/042055, relating to apparatus and methods for thermally treating webs which comprise thermoplastic respectively meltable compounds, thereby creating cylindrical or elliptic consolidation regions by employing a thermal energy source, such as ultrasonic energy, as well as to webs comprising elliptic consolidation regions. In a particular aspect the invention concerns apparatus and methods for creating the consolidation regions by using an anvil with a flexible elongated member.

The principles of this technology are furthered and applied to a particle loaded web, see WO2014/001487 (C4S). Therein, a fibrous matrix comprises meltfusionable material as well as particulate material, such as superabsorbent polymer particles. The particle loaded structure is bonded by a meltfusion bond point pattern, which is preferably created by ultrasonic welding.

It is also well known to bond webs with treatment rolls or cylinders, that have a patterned surface, such as irregular, often aesthetically pleasing patterns, or regular patterns comprising pins or protrusions extending from the surface, see e.g. EP1144187, disclosing a bonding roll with pins with a spherical pin head. Generally, such bonding rolls achieve the bonding by melt fusion bonding, and the energy is provided via heating and/or applying ultra-sonic energy. All these bonding rolls have in common that they exhibit a low deformation such that a sharply defined compression can be achieved.

However, the rigidity of the bonding tools results in non-satisfactory bonding of particle loaded webs, in particular for higher particle basis weights, and/or at higher process speeds, as the particles are detrimentally present in the bonding regions.

Thus, there exists a need to create particle free regions in a particle loaded web for example to allow to position other substances which may be incompatible with the particles in this regions and/or to bond webs in these particle free regions adhesively or by meltfusion bonding, in particular in the context of employing low deformation treatment tools, such as bonding tools for a broad range of bonding techniques such as ultrasonics but also heat or pressure embossing as well as glue or adhesive based bonding.

### Summary

In a first aspect, the present invention is a continuous operation apparatus, as defined in claim 1, for creating particle free regions in a particle loaded fibrous web. The apparatus exhibits in Cartesian coordinates a machine direction (MD) defining the path of the fibrous web, a cross-machine direction (CD) perpendicular thereto and a z-direction perpendicular to both MD and CD. The particle loaded fibrous web exhibits in Cartesian coordinates length direction, generally aligned with MD, a width direction, generally aligned with CD, and a thickness direction, generally aligned with the z-direction. The particle loaded fibrous web comprises an aggregation of fibers, a plurality of particles positioned in interfiber interstices of the web or on the x-y-extending surfaces of the web.

The apparatus comprises a first and a further web guide. The first web guide is a guide roll adapted to move around an axis essentially aligned in CD of the machine, and further exhibits a radial coordinate r, aligned with the z-direction of the web when the web is in tangential positioning to the guide roll, and an angular coordinate Φ, aligned with the x-direction of the web when the web is in a tangential positioning to the guide roll, wherein the orientation of both the +x- and the +Φ -direction correspond to the orientation of the MD-direction upon movement of the web during operation of the apparatus. The guide roll further comprises a guide roll surface, a plurality of protrusions positioned on and extending r-directionally from the surface of the web guide roll, and a plurality of particle displacer tools, wherein a particle displacer tool is positioned on and extending r-directionally from the surface of the web guide roll, and positioned +Φ -directionally relative to a protrusion.

The first and further web guides form a gap adapted to receive the web between the surface of the guide roll and the further web guide, and exhibiting a gap width aligned with the z-direction of the web.

A protrusion comprises a protrusion base oriented towards the axis of the guide roll, a protrusion apex corresponding to the portion that extends r-directionally most outwardly away from the axis of the guide roll, and a protrusion stem connecting the protrusion base and the protrusion apex.

A particle displacer tool comprises a displacer tool base oriented towards the axis of the guide roll, a displacer tool apex corresponding to the portion that extends r-directionally most outwardly away from the axis of the guide roll, and a leading ridge that extends from the displacer tool base to the displacer tool apex along the most forwardly positioned points of the displacer tool in the +Φ -direction at any radial position, wherein a projection of the leading ridge on the Φ direction that is longer than the projection of the displacer tool on the cross-direction.

The protrusion apex exhibits a flexibility in the -r direction of less than 30 µm, preferably less than 10 µm and more preferably less than 5 µm at a load of 200 N applied -r-directionally to the apex.

A protrusion may be essentially integral with the guide roll, a particle displacer tool may be essentially integral with the guide roll, and a particle displacer tool may be essentially integral with a protrusion.

The apparatus further comprises bonding elements selected from the group consisting of an energy supply to enable melt fusion bonding, preferably selected from the group consisting of heating, pressurizing, applying ultrasonic energy and of an adhesive supply, preferably to the apex of a protrusion.

In an apparatus according to the present invention, the plurality of protrusions may form a protrusion pattern, wherein a straight line connecting neighboring protrusions forms preferably an angle of more than 0°, more preferably more than 5° or even more preferably more than 10° to the Φ - direction of the guide roll.

In another aspect the present invention is a process for creating particle free regions in a particle loaded fibrous web on an apparatus for continuous operation according to any of the claims 1-3. The process comprises the steps of
- providing
   a) a particle loaded fibrous web comprising an aggregation of fibers and a plurality of particles positioned in interfiber interstices of the web or on the x-y-extending surfaces of the web,
   b) a first and a further web guide,
      the first web guide being a guide roll exhibiting a radial coordinate r, an angular coordinate Φ, aligned with the machine direction of the apparatus
      when the web is in a tangential positioning to the guide roll, and a cross-direction perpendicular to the machine direction;
      wherein the guide roll further comprises
      a guide roll surface, a plurality of protrusions positioned on and extending r-directionally from the surface of the web guide roll, and a plurality of particle displacer tools positioned on and extending r-directionally from the surface of the web guide roll;
   c) wherein the web guides forms a nip between the surface of the guide roll and the further web guide, adapted to receive the web and exhibiting a nip width;
- feeding the particle loaded web into the nip, whilst concurrently rotating the guide roll such that the displacer tools penetrate into the web prior to the protrusions.
thereby dislocating particles cross-directionally and creating a particle-free region corresponding to the apex of the displacer tool when the apex is in its point of culmination relative to the further web,
wherein the apex of the protrusion dislocates less than 30 µm, preferably less than 20 µm and even more preferably less than 10 µm in the -r direction.

The particle loaded fibrous web may comprise thermoplastic material, and the process may further comprise the step of creating meltfusion bonding in the particle free region by applying energy selected from the group consisting of pressure, heat, sonic, and ultrasonic energy.

The process may further comprise the step of applying a further material to the particle free regions, wherein preferably the further material is a treatment fluid, optionally a liquid bonding agent, preferably a hot melt adhesive. The particle loaded web may further comprise non-thermoplastic material and/or comprise sub-webs.

The process may be employed for forming a liquid absorbent structure, wherein the particle loaded fibrous web is liquid absorbing and the particles are preferably superabsorbent polymer particles. The process may further be employed forming absorbent articles.

### Brief description of the Figures

Fig. 1A, B, and C depict schematically a web, a particle loaded web and a particle free bond region in a particle loaded web, respectively.
Fig. 2 depicts schematically an equipment and process set up according to the present invention.
Fig. 3A and B show schematically an exemplary arrangement of a protrusion and a displacer tool according to the present invention.
Fig 3C depicts schematically cross-sectional views of a particle displacer tool, indicating various flank designs.
Fig. 4 depicts schematically the processing of a particle loaded web in a process according to the present invention and a corresponding equipment set up.
Fig 5 depicts schematically a glue supply system for application in the present invention. Same numerals refer to same or equivalent features.

### Detailed description

The present invention is a process for creating particle free regions in a particle loaded web and an apparatus for operating such a process. Within the present context, the expression "creating particle free regions" refers to relocating particles out of these regions whilst the web remains to be present in these region. Thus, to illustrate this for a particle loaded web exhibiting an essentially homogeneous distribution of particles throughout its fibrous matrix prior to the treatment according to the present invention or prior to being run through the apparatus according to the present invent, the particle loaded web comprises essentially particle free regions thereafter.

A particle loaded web according to the present invention comprises fibers forming a fibrous matrix.

"Fiber" refers to a continuous or discontinuous member having a high ratio of length to diameter or width. Thus, a fiber may be a filament, a thread, a strand, a yarn, or any other member or combination of these members. The term "fibers" also includes fibers, which over their length appear partly molten (such as when a fiber extends through a bonding region) or which are partly molten across the cross-section of the fibers, such as when a fiber is attached only superficially whilst maintaining its fibrous shape, such as when the sheath material of a bicomponent fiber is molten, but the fiber core material not.

Suitable fibers may be limited length fibers, such as knows as staple fibers, but also natural fibers like cellulose fibers. Typically such natural or staple fibers exhibit a fiber length from about 0.1 to 15 cm. often from about 2 to 7 cm. Suitable fibers may be substantially continuous fibers, which are not cut from their original length prior to being formed into the fibrous matrix. Substantially continuous fibers may have average lengths ranging from greater than about 15 cm to more than one meter.

Fibers useful for the present invention may be natural ones, such as modified or unmodified cellulose fibers, or synthetic ("man made") fibers including fibers as made from natural ingredients such as without limitation viscose / rayon.

In a first aspect, fibers useful in the present invention may be non-thermoplastic. Such fibers may, for example, very suitably be bonded by adhesives or glues which may be applied to the particle free regions. The selection of such adhesives or glues is not particularly limited. Preferably, such glues are applied in a liquid form to the particle region, and solidify and/or develop their adhesive properties by cooling or any other treatment. A particular glues may be of the well-known hot-melt type, which may be selected specifically for various applications.

In another aspect of the present invention, the fibers comprise thermoplastic material allowing for meltfusion bonding. Such fibers can be made from a single polymer (monocomponent fibers), or can be made from more than one polymer (e.g., bicomponent fibers). As used herein, the term "bicomponent fibers" refers to thermoplastic fibers that comprise a core fiber made from one polymer that is encased within a thermoplastic sheath made from a different polymer. The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer. Suitable bicomponent fibers can include sheath/core fibers having the following polymer combinations: polyethylene / polypropylene, polyethylvinylacetate / polypropylene, polyethylene / polyester, polypropylene / polyester, copolyester/polyester, and the like.

Spunmelt fibers are made into a fibrous matrix in one continuous process. Fibers are spun and then directly dispersed into a fibrous matrix by deflectors or can be directed with air streams thereto. It should be noted that the term "spunmelting" includes different methods such as spunbonding, meltblowing, or advanced meltblowing, referring to techniques combining elements of spunbonding and meltblowing (see e.g. BIAX ® fibers). Spunmelting may provide essentially endless fibers, such as resulting from the spunbonding process, or fibers exhibiting a limited length, such as resulting from the meltblowing process with a fiber length of typically 30 mm to about 100 mm or more. In particular when the resulting structure is further incorporated in articles which undergo significant compression, such as during transportation and storage, it is preferred that the fibrous matrix comprises resilient materials, which also exhibit low tendency of "cold flow". Preferred materials comprise polyester and/ or co-polyester. Optionally the structure may comprise different types of fibers in a mixed or in a layered arrangement.

The fibers useful in the present invention may generally have a thickness ranging from about less than about 1 to over 15 dTex. Mostly the dTex of the fibers will range between about 0.5 and 7, though finer or even nano-fibers may be included.

Suitable fibers may be crimped and have a crimp count of at least two crimps per centimeter and a percent crimp of at least about 15%, preferably at least about 25%. Percent crimp is defined as the difference between the uncrimped length of the fiber (measured after fully straightening a sample fiber without stretching it elastically or plastically) and the crimped length (measured by suspending the sample fiber with a weight attached to one end equal to 2 mg per decitex of the fiber, which straightens the large-radius bends of the fiber) divided by the crimped length and multiplied by 100. Crimped fibers may be staple fibers or result from a process such as spunmelting.

For executing the present invention it is not particularly relevant how the fibrous matrix is formed, as long as the criteria as defined herein are met. Thus the fibers may be laid down to form a fibrous matrix in-line, i.e. during a continuous process during which the absorbent matrix is formed. Additionally or alternatively, the fibrous matrix may be provided as a preformed and/ or prebonded web.

The term "web material" refers to a material, which is essentially endless in one direction, i.e. the longitudinal extension, or the length, or the x-direction in Cartesian coordinates relative to the web material. Typically, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web.

The web may be a "nonwoven" material that is a manufactured sheet or web of directionally or randomly oriented fibers as described in the above. The nonwoven may be consolidated and pre-bonded together by friction, such as resulting from hydroentangling or needlepunching, cohesion, adhesion or meltfusion bonding, such as between connecting points of neighboring fibers molten by air-trough bonding, or one or more patterns of bonds and bond impressions created through localized compression and/or application of pressure, heat, ultrasonic or heating energy, or a combination thereof. Preferably the web is not overly bonded but allows a certain degree of dislocating of the fibers relative to each other. The basis weight of nonwoven fabrics is usually expressed in grams per square meter (g/m²).

The fibrous matrix may also be provided by fabrics which are woven, knitted, or stitch-bonded with yarns or filaments.

The selection of particles useful in a particle loaded web according to the present invention is not particularly limiting, provided the particles match the fibrous web such that at least a portion of the particles may be positioned in the interfiber interstices of the web.

Thus suitable particles matching a broad range of the web materials as described in the above may have a particle size distribution typically ranging from few micrometer, often more than 50 µm to few millimeter, often less than about 1500 µm. Often their particle size range will be between about 50 µm and about 800 µm. Particles may have a regular shapes, such as approximately spherical, or may have an irregular shape, such as when larger particles are ground or otherwise broken up. Particles may be aggregates of or with smaller sized particles, preferably exhibiting sufficient aggregation strength to not break up during the present process.

The particles may be have essentially the same composition, or there may be a mixture of different particulate materials. Without any intention for a limitation, particles may be activated carbon, such as for filter applications, food ingredients, such as for the preparation of packaged food, or seeds or fertilizer for horticultural applications.

In a particular application, the present invention may be employed for absorbent structures, and the particles may be so called superabsorbent polymers. Superabsorbent polymers (SAP) are often also referred to as "super absorbent", "super absorbent material", "absorbent gelling material", hydro gel, or "absorbent polymer material", or "AGM", all referring to partially cross-linked polymeric materials, which can absorb water whilst they are swelling to form a gel.

Suitable superabsorbent materials useful in the present invention include superabsorbent particles as are known in the art, and may be formed from organic material which may include natural materials such as agar, pectin, and guar gum, as well as synthetic materials such as synthetic hydrogel polymers. Synthetic hydrogel polymers include, for example, carboxymethylcellulose, alkali metal salts of polyacrylic acid and its copolymers, and the like. The superabsorbent polymers are typically lightly crosslinked to render the material substantially water insoluble. Crosslinking may, for example, be by irradiation or by covalent, ionic, Van der Waals, or hydrogen bonding. Suitable materials are available from various commercial producer or vendors, such as the Nippon Shokubai KK (Himeji, Japan), Evonik-Stockhausen GmbH (Marl, Germany), and BASF SE (Ludwigshafen, Germany), Danson (Yixing, China), EKOTEC GmbH (Haan, Germany) and many more.

Such Superabsorbent Polymer particles preferably exhibit a particle size of between 50 and 1500 µm, Often, the median particle diameter ranges between about 200µm and about 800 µm.

A particle loaded web suitable for the present invention is a matrix or aggregate of fibers with particles positioned at least in interfiber interstitials.

The particle loaded web may have a broad range of basis weights, as a combination of he basis weights of the fibers and the particles, though other ingredients, such as adhesives, may be added.

The basis weight of the fibers may range from values as low as few gram per square meter to well over 1000 g/m². Often, the fibrous basis weight will be more than 5 g/m² or even more than 15 g/m² but less than about 500 g/m² or even less than 200 g/ m². The densities of the fibrous matrix, i.e. excluding particles, may range from as low as 2 kg/m³ to 1000 kg/m³, often materials will be in the range of from about 20 kg/m³ to about 400 kg/m³.

The particle basis weight may range from as low as few gram per square meter to several hundred gram per square meter. Often, the particulate basis weight will be more than about 10 g/m² more than about 50 g/m² or even more than about 80 g/m². Often, the particulate basis weight may be less than about 500 g/m².

Optionally the particles may be homogeneously distributed throughout a particle loaded web. Alternatively, particles may show a predetermined distribution both with regard to local particle density, including certain regions in the web that are essentially free of particles, but also with regard to varying particle properties, such as particle size distribution. For certain executions, it may be advantageous to have particle segregation, as may occur when a plurality of particles exhibiting a particle size distribution is spread over the surface of a fibrous matrix. Then, for example, smaller particles may penetrate deeper or even through the fibrous matrix, medium sized particle may penetrate into interfiber interstices, and larger particles may even rest on the surface.

Often though not necessarily, the particles are essentially unbonded to the fibers, but only mechanically entrapped in the interstices. If there is some bonding between the particles or between the particles and the fibers, this should not be overly strong and allow for dislocating the particles relative to their original position.

A particle loaded web useful for the present invention may be formed by a variety of processes, which is not critical as long as the above mentioned requirements are satisfied. Thus a particle loaded web may be preformed in a separate process and then be supplied in a roll stock form, such as on a roll, a spool, or "festooned" in boxes. The particle loaded web may be formed in an essentially continuous process combined with the process according to the present invention, such as when loose, individualized fibers are mixed with particles, or particles are applied to a preformed web.

A particle loaded web may also comprise a stratified arrangement of several sub-webs, such as when a first fibrous aggregate is combined with one or more sub-webs to form a sandwich structure. These one or more sub-webs may differ in their composition, such that one sub-web may have particles in its interfiber interstices, another one may be essentially particle free, or particles may form a particle layer between two sub-webs. Within the overall limitations as described in the above, there are many possible combinations for forming a particle loaded web.

The present invention is an apparatus and a process for creating particle free regions in a particle loaded web by dislocating particles.

Thus it relates to continuous operation apparatus, which exhibits in Cartesian coordinates a machine direction (MD) defining the path of the fibrous web, a cross-machine direction (CD) perpendicular thereto and a z-direction perpendicular to both MD and CD.

The apparatus is adapted to run a particle loaded fibrous web and to treat it, whereby the length direction of the web is generally aligned with MD, the width direction generally aligned with CD, and the thickness direction, generally aligned with the z-direction, of the continuously operating machine, respectively.

The apparatus comprises a first and a further web guide, which form a gap into which the web may be guided.

A first web guide is most preferably a guide roll, though a continuous belt system is contemplated to function equivalently. Whilst the following explanation is focusing on a guide roll, a skilled person can readily re-apply the teaching to such a continuous belt system.

Such a guide roll is adapted to rotate around an axis which is essentially aligned in CD of the machine, i.e. the axis orientation should not deviate more than 15°, preferably not more than 5° from the CD of the machine.

The guide roll can also be described by using a cylindrical coordinate system with an axial coordinate parallel to the cross-machine direction of the machine, a radial coordinate r running from the axis outwardly and an angular coordinate Φ circumferentially. The orientation of Φ is such that upon rotation of the guide roll in normal operation, the +Φ-direction is aligned with the MD of the equipment, respectively the x-direction of the web moving through the gap between the guide roll and the further web guide.

The first guide roll further comprises a guide roll surface, a plurality of protrusions positioned on and extending r-directionally from the surface of the web guide roll, such that the surface can also be seen as depressions between the protrusions, and a plurality of particle displacer tools. A displacer tool is positioned on and extending r-directionally from the surface of the web guide roll and positioned +Φ -directionally before a protrusion.

A protrusion as can be useful in context of the present invention is generally known in the art for embossing of nonwovens or wipe materials and comprises a protrusion base oriented towards the axis of the guide roll, a protrusion apex corresponding to the portion that extends r-directionally most outwardly away from the axis of the guide roll, and a protrusion stem connecting the base and the apex, which shape is of secondary importance in the context of the present invention, although for ease of manufacturing, the stem may be represented by flanks directly connecting the apex and the base.

If, for example and without intending any limitation, both the apex and the base have a rectangular shape, the stem may have planes connecting the apex and the base, such that the protrusion has a shape of a trapezoidal prism. If both the apex and the base have the form of a circle or an ellipsis, the protrusion may take the shape of a truncated cone. The base and the apex may also be cross - or Φ-directionally offset, such that the protrusion takes the shape of an oblique cylinder. It is, however, also contemplated and within the scope of the present invention that at least the apex of a protrusion has a more complex form, so as to allow for ornamental shapes of the bonding regions.

Protrusions may have a protrusion height from the base to the apex which can be as low as 2 mm or even less, and often will be more than 3 mm , or even more than 5 mm, and may even be more than 10 mm for certain applications.

The apex exhibits preferably an elongated shape, i.e. it exhibits a larger extension in machine (or Φ -) direction than in cross-machine direction.

The surface area of the apex, as determined as a projection of the apex onto the surface of the guide roll, should be sufficiently small such that particles can be displaced without crushing them, and without tearing the fibers surrounding and holding them. Thus, the apex surface area may be as much as over 8 mm² or even 10 mm² for elongated apex shapes, or be as small as 2 mm² or even less than 1 mm².

A protrusion may be formed integrally with the guide roll or at least with the material of the surface of the guide roll, e.g. when a surface shell is mounted on a guide roll core, as well known in the art. Within the context of the present invention, the term "integral" refers to two or more features being made of essentially the same material, such as when protrusions are shaped from a cylinder by removing material between the protrusions, such that the protrusions form "islands" in "sea" of depressions.

A protrusion is adapted to compress the web in the gap. Whilst the web may be and often is somewhat compressed between the guide roll surface and the further web guide in regions other than the ones corresponding to the protrusions, the web is much more compressed by the protrusions, for example to form bonding regions in the web.

For such an applications, and optionally combined with added energy such as heat, pressure, or sonic respectively ultrasonic energy, the fibers of the web may be compressed such that interstitial voids are eliminated and actually melt fusion bonds may be formed. If combined with glue or adhesive, as will be discussed in more detail herein below, there may be bond points formed in the web in the region corresponding to the protrusions when such a glue or adhesive fill the small remaining interstices in the compressed regions or on the surface in these regions.

The plurality of protrusions typically forms a protrusion pattern, corresponding to an overall bonding pattern which typically correlates closely with the pattern of bonding regions in a bonded web.

The overall protrusion pattern may comprise sub-patterns, such as when a sub-pattern of protrusions of a certain shape is combined with another sub-pattern of protrusions of a different shape.

The protrusion pattern or sub-pattern may be a very open regular pattern and exhibit one protrusion for 200 mm² or more, often between 100 mm² and 200 mm². For smaller protrusion apex surface areas, e.g. 0.5 mm², it may exhibit one protrusion for as low as 20 mm² and for larger protrusion apex areas, e.g. 8 mm², it may exhibit on protrusion for 50 mm².

Often it may be advantageous, if the protrusions are arranged in a pattern that is not exactly aligned machine (or Φ -) direction, but that a straight line connecting neighboring protrusions forms an angle of more than 0°, preferably more than 5° or even more preferably more than 10° thereto.

The protrusions may also show an irregular protrusion pattern that may impart an aesthetically pleasing impression to the web, see e.g. US2015/0087670 (P&G), or US2015/0001783 (P&G).

The overall protrusion apex area, i.e. the sum of the surface areas of all apexes, may cover as little as less than 0.025% of the guide roll surface, often less than 1%, but for certain applications over 20%, often over 15% of the guide roll surface area. When the present invention is combined with a bonding step, such protrusion apex areas will generally, though not exactly, mirror the bonding area percentage.

A guide roll suitable for the present invention further comprises a displacer tool, i.e. a particle displacer tool. A displacer tool is adapted to displace particles in the particle loaded web so as to be removed from predetermined regions of the web before these are highly compressed, e.g. to allow application of other materials such as glues or to ease bonding. In general terms, the displacer tool moves particles laterally away (e.g. left and right relative to the machine (or Φ -) direction of the machine or guide roll, respectively). The fibrous material should exhibit sufficient integrity to leave at least a portion of the fibers not laterally dislocated. The particle displacer tool operates essentially at matched speed to the particle loaded web in machine (or Φ -) direction, and it gradually penetrates through a surface into the web, thereby moving the particles as encountered during this movement primarily cross-directionally away and out of its path, though a z- or r-directional movement may occur simultaneously.

A particle displacer tool comprises a displacer tool base oriented towards the axis of the guide roll, a displacer tool apex corresponding to the portion that extends r-directionally most outwardly away from the axis of the guide roll, a leading ridge that extends from the displacer tool base to the displacer tool apex along the most forwardly positioned points of the displacer tool in the +Φ -direction (each at a given radial position), and displacer tool flanks extending from the leading ridge towards base or apex.

As the displacer tool rotates with the guide roll towards the gap between guide roll and further web guide whilst the web runs into the same gap, a displacer tool gradually approaches the surface of the web, penetrates gradually into the web until the highest compression zone is reached at the gap width. When the displacer tool penetrates into the particle loaded web at matched x- or +Φ directional speed, and encounters a particle with its ridge or flank, the particle is moved laterally away without the need for machine-directional de- or acceleration.

Thus, in the "wake" of the displacer tool a particle free zone is created, in which for example a protrusion for forming a particle free bonding region may very suitable be positioned.

The relative positioning of a displacer tool and a protrusion is such that the protrusion is positioned in this "wake" of the displacer tool, i.e. Φ-directionally behind the displacer tool. Preferably, each protrusion is preceded by a displacer tool, although in particular for protrusion patterns with varying protrusion shapes, certain protrusions may not have a corresponding displacer tool.

A displacer tool may be executed in a wide variety of shapes and forms, as long as the lateral displacement of the particles takes place. Also, the r-directional as well as the cross-directional extensions should be adapted to the shape of the corresponding protrusion. Whilst the ridge may be a straight line, it is preferred that the ridge has a curvature, and even more preferably the curvature is such that its gradient dr/dΦ decreases from the most forward point of the ridge towards the apex of the particle displacer tool. In a particular execution, the ridge may correspond to a sector of a circle or an ellipsis. This ensures gentle displacement operation as the r-directional speed component of which exerts a forcer on the particles and the fibers decreases with increasing compression of the web.

Preferably, the cross-directional extension matches the cross-directional extension of the protrusion, though a greater width of the displacer tool may be acceptable. Similarly, and in particularly if the protrusion exhibits a forward (+Φ-directional) ridge and its shape is tapering cross-directionally outwardly to its largest cross-directional extension, the displacer tool may have a narrower cross-directional extension than the protrusion.

Preferably, though not necessarily, the particle displacer tool exhibits a symmetric shape to a MD or Φ -directionally extending line.

Preferably, the height of the displacer tool, i.e. the r-directional level of the displacer tool apex, matches the height of the protrusion, though certain deviation is acceptable. Preferably, the displacer tool is positioned close to the protrusion, though a certain spacing is acceptable. Optionally, the rearward (-Φ -directionally) shape of the displacer tool may be adapted to conform to the shape of the protrusion. Optionally, the displacer tool may be integral with the protrusion. Optionally the displacer tool may be integral with the guide roll or at least the surface thereof. Optionally, the displacer tool and the protrusion may be integral with each other and with the surface of the guide roll.

Optionally, the ridge may be chamfered or beveled, such as when it was made with a sharp edge that has been beveled, and the ridge actually takes the shape of a flat plane of or several smaller flat planes. The skilled reader will also readily appreciate, that when referring to "gradual" or curved shapes a polygonal or polyplanar shape will provide an equivalent functionality.

A ridge may have the same -Φ-directional extension (i.e. its projection onto the surface of the guide roll) as the protrusion. Preferably this is more than twice or ten times or even higher the cross-directional extension of the protrusion.

As the skilled reader will readily appreciate, the displacer tool will follow a radial movement and gradually penetrate into the tangentially moving particle loaded web. Thus, even a relatively steep slope between the ridge and the surface, i.e. up to a right angle but preferably not more, the initial contact with the web will be at a smaller angle between the ridge and the web surface, thereby easing penetration and lateral displacement of the particles.

The flanks of the particle displacer tool extend from the ridge towards the base as well as to the apex and may take a broad range of shapes as long as the path of the particles for a lateral displacement is not obstructed. Thus, any cross-section may as long as particles are not prevented from being moved cross-directionally. Preferably, the ridge of the displacer tool gradually transitions into the flanks of the displacer tool, though beveled transition may be well acceptable.

A displacer tool may terminate with or Φ-directionally before the protrusion. It may, however, also extend -Φ-directionally over the protrusion, such as when covering the apex of the protrusion. For such an execution, the requirements and options for the protrusion, such as protrusion height, apex shape or area, or rigidity shall be read to include at least the covering portion of the displacer tool.

The further web guide may also be executed as a further guide roll, though a belt system may provide equivalent functionality of pressing the web against the first web guide. Often, the further web guide exhibits a smooth surface, such as when in a particular execution the further web guide is executed as a sonotrode, preferably a rotatably mounted sonotrode, of an ultrasonic bonding system, although the further web guide may have protrusions of various shapes. For the latter, the protrusions of the further web guide may intermeshingly engage with the protrusions of the guide roller, or may be arranged and operated such that the web is compressed between the protrusions. Optionally, also the protrusion of the further web guide may be preceded by particle displacer tools, such that both the protrusions of the guide roller and of the further web guide created particle free regions. The present invention is particularly beneficial when applied in the context of a compression tool exhibiting small deformation in the -r-direction. This should be seen in contrast to a "flexible anvil" tool, such as described in WO2012/042055 (C4S) or WO2014/001487 (C4S) in the context of ultrasonic bonding.

Without wishing to be bound by the theory, it is believed that for flexible bonding tools and in particular in the context of ultrasonic bonding the flexibility of the anvil allows for the creation of particle free bonding regions even in case of high particle basis weights. However, the use of such flexible elements not only reduces the accuracy of the bonding regions, but also creates limitations, both with regard to the shape of the formed regions, such as when a pattern with non-elliptical shape or larger distances of small protrusions are required, as well as to the applications, for example if- partly due to the mentioned reduced accuracy of the operation of the flexible tools - perforations or apertures are unacceptable. The present apparatus respectively the present process not only provide an increased accuracy, but also allows for a broader selection of protrusion shapes and even - in case of bonding - even for alternative bonding options, such as glue bonding. This is of particular interest if materials are used which are not compatible with ultrasonic bonding requirements.

Thus, the present invention relates particularly to process wherein a protrusion dislocates r-directionally towards the axis by less than 30 µm, preferably less than 20 µm and even more preferably less than 10µm. Accordingly, the present invention relates to an apparatus comprising protrusions, that exhibit a r-directional stiffness as may be expressed by the protrusions dislocating -r-directionally by less than 50µm, preferably by less than 25 µm or even more preferably by less than 10 µm under a load of 200 N applied to a protrusion.. In a process according to the present invention, a particle loaded web - be it preformed or in-line formed prior to the presently described step - is fed into a gap between to web guides. At least one of the web guides is executed as web guide roll, as described in the above, comprising protrusions and particle displacer tools. The gap may be dimensioned relative to the web such that it exhibits a gap width that is slightly smaller than the thickness of the particle loaded web before the present process step, so as to moderately compress the web in this process step also in other regions than the compression regions created by the protrusion, though it may be - and often is - slightly larger than the web thickness to not significantly impact the web in the non-compressed regions.

The protrusions extending into the gap exhibit a size such that the smallest distance between the apex of the protrusion and the counteracting further web guide, the "protrusion apex gap width" is small or even zero.

"Small" may be described relative to the amount of fibers present in this compression zone, such that this distance is about the ratio of the basis weight of the fibers divided by the (bulk) density of the material. Thus, for example for a polyester web, exhibiting a polymer density of about 1400 kg/m³, at a basis weight of 10 g/m², the protrusion apex gap width should be about 14 µm or more, if no perforations should be created in the compression zone. Otherwise, the protrusion apex gap width may be even further reduced, and be close or even equal to zero.

The present invention is particularly useful for the bonding of particle loaded webs. Such bonding may be achieved by melt fusion bonding, if appropriate meltfusionable material is present in the particle loaded web. The melt fusion bonding may be achieved by a conventional means, such as applying thermal energy, pressure, or (ultra-)sonic energy.

In a particular execution, adhesives may be applied through a glue feeding conduit from the guide roll through the protrusion to an opening in the protrusion apex, where the glue may be applied very cleanly and accurately dosed to the web. A skilled person will readily realize that for this execution the protrusion apex gap width should be sufficiently large to not induce melt fusion bonding, but to leave the fiber interstices to receive small amounts of glue.

A particular area, where the present invention can be applied very beneficially is the manufacturing of absorbent structures for absorbent articles like baby or adult incontinence diapers, absorbent underwear, feminine hygiene articles or the like. Such articles typically comprise a wearer oriented surface with a liquid permeable topsheet, a liquid impermeable backsheet oriented away from the wearer and an absorbent structures positioned there between. Such articles as well as other features and elements of such articles like fastener or closure element, or fit improving elements, are well known in the art.

### Particular executions

In the following, various particular executions of the present invention are described, which however should not be seen limiting in any way, and which may also be combined with each other.

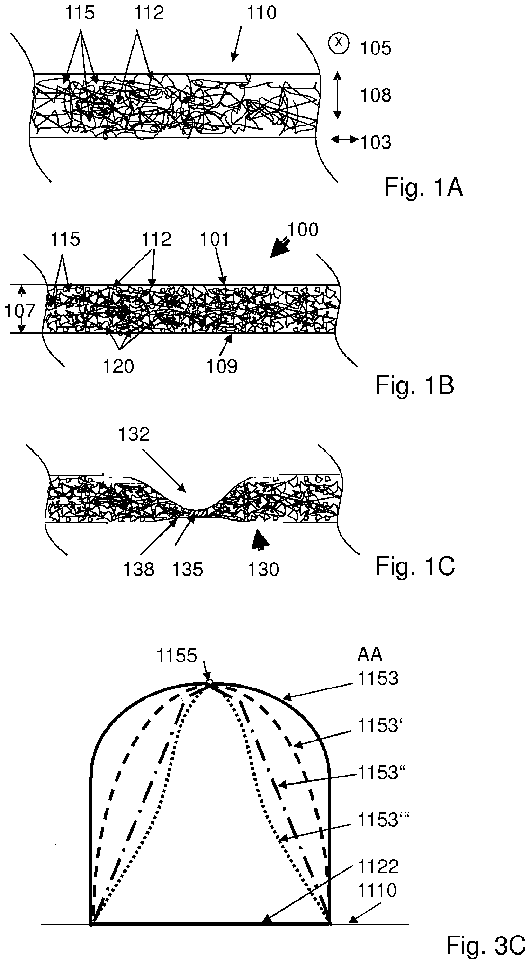

Referring to Fig. 1A, an exemplary execution for a fibrous matrix or web 110 is shown schematically, comprising fibers 118 and interfiber interstices 115. The matrix exhibits in its web-coordinate system a thickness dimension 108, a width dimension 105, and a length dimension 103.

Fig. 1B shows the same web loaded with particles 120 positioned in interfiber interstices 115 to form the particle loaded web 100. Whilst not shown, particles may also be positioned on a first surface 101 and an opposite surface 109. Also not shown in the figure are optional further webs as may be positioned in a facing relationship to these surfaces.

Fig. 1C depicts, also schematically, a bonded particle loaded web 130 after it went through the treatment according to the present invention and a bonding step. Thus, the bonding indentation, as may extend from one of the surfaces only, or as shown in Fig. 1C both from the first surface forming a first compression indentation 132 and from the opposite surface forming a second compression indentation 138, is essentially free of particles. In the center of the indentation(s) the fibers may be bonded in the bonding region 135 such as by meltfusion bonding or by glue bonding.

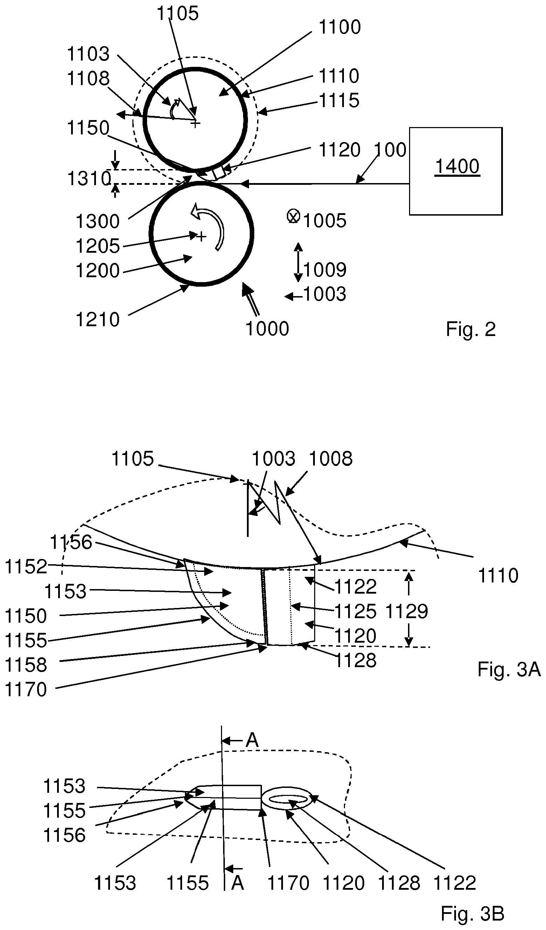

Figure 2 depicts schematically the key process set up and the apparatus 1000 according to the present invention. The apparatus exhibits a machine direction 1003, a cross-machine direction 1005 and a thickness direction 1008, which are aligned with the length, width, and thickness dimensions of the particle loaded web, respectively.

A particle loaded web 100 - with its thickness not shown to scale - is supplied by a supply unit 1400. The particle loaded web may be prepared inline in the supply unit or it might be prepared off-line and be transported to the supply unit.

The particle loaded web 100 is transferred towards the apparatus 1000 for creating particle free regions by dislocating particles.

The apparatus comprises a guide roll 1100 and a counteracting web guide, here shown also as a roll 1200, which may be executed as a rotary sonotrode of an ultrasonic system.

The rolls are rotatably mounted to allow the particle loaded web to move though gap 1300, formed between surface 1110 of the guide roll 1100 and surface 1210 of the further web guide 1200, and exhibiting gap width 1310. The guide roll exhibits a guide roll axis 1105 along the cross-direction of the roll, which is aligned with the cross-direction of the apparatus. The guide roll further exhibits a radial coordinate r 1108 and a circumferential coordinate Φ, 1003.

Also shown in Fig. 2 is a protrusion 1120 and a corresponding particle displacer tool 1150. For ease of representation, only one of each is indicated, though there is a plurality of such protrusions and corresponding particle displacer tools, indicated by outer circumscribing circle 1115.

In Fig. 3, an enlarged view of a protrusion 1120, with Fig. 3A showing a side view and Fig. 3B a top view. Fig. 3C shows a cross-sectional view AA through a particle displacer tool. Indicated in Fig 3A is a coordinate system for the guide roll with an r-direction 1108 extending away from the axis 1105 and a circumferential axis Φ 1103 aligned with the surface 1110 and oriented such that it is aligned with MD 1003 of the apparatus and the length direction 103 of the web in the gap 1300.

Protrusion 1120 exhibits a protrusion height 1129, a protrusion base 1122 and a protrusion apex 1128, connected by the protrusion stem 1125. As exemplarily indicated, the protrusion exhibits an elliptic cross-section both at the base and at the apex, and the longer axis of the ellipse is generally MD- or Φ -directionally oriented.

Particle displacer tool 1150 is positioned Φ -directionally before the protrusion and exhibits a particle displacer base 1152, apex 1158, ridge 1155 and flanks 1153. Fig. 3C shows a cross-sectional view of a displacer tool, with the flank 1153 having a rounded portion towards the ridge 1155 and a straight vertical portion towards the base. Also exemplarily indicated are other shapes for a flank design, namely a fully rounded flank, indicated by dashed line 1153', a flank represented by two straight plane portions, see dash-dotted line 1153", or a bell shaped cross-sectional line, see dotted line 1153"'.

As indicated for the specific execution, the protrusion and the particle displacer tool are not integral but separated by a spacing 1170.

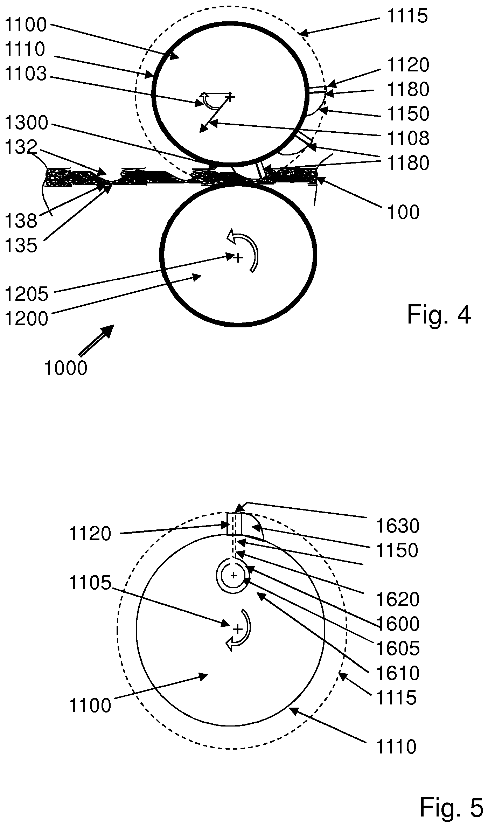

In Fig. 4, a particle loaded web 100 is shown as it runs through an apparatus according to the present invention.

Three combined units 1180, each made up of a protrusion 1120 and a particle displacer tool 1150 are shown in order to depict how the leading portion 1156 of the ridge gradually penetrates into the particle loaded web and also gradually "ploughs" the particles cross-directionally away.

Also indicated in Fig. 4, a bonding step may concurrently be executed, such as by applying energy to the web via heated protrusions or via applying ultrasonic energy, if the further roll be executed as a rotatably mounted sonotrode. This may create first and second compression indentations 132 and 138, respectively, and therein a bonding region 135.

In Fig. 5, a glue application system combined with a particle displacer apparatus according to the present invention is schematically depicted.

In a cross-sectional view of a guide roll 1100, a glue supply system 1600 for application of glues like hot melts is indicated, comprising a glue supply 1610 that may run cross-directionally through the guide roll comprising a slot bushing 1605, and a glue conduit 1620 connecting the glue supply 1610 with a glue orifice 1630 positioned at the apex of a protrusion. Upon rotating of the slot bushing 1605 a small amount of glue is released from the glue supply 1610 to the glue conduit, allowing very easy and clean application of small amounts of glue directly to the tip of the protrusions.

### Methods

For determining surface area of the protrusion and/or the geometry of the particle displacer tools, and in particular for determining the bonding line area of a bonding pattern an image analysis tool as available as ImageJ software (ver. 1.48, National Institute of Health) or equivalent and as described in more detail in US2015/0086760 under the section "Bonding pattern analysis".

## Claims

1. A continuous operation apparatus for creating particle free regions in a particle loaded fibrous web,
said apparatus exhibiting in Cartesian coordinates
a machine direction (MD) defining the path of the fibrous web,
a cross-machine direction (CD) perpendicular thereto and
a z-direction perpendicular to both MD and CD;
wherein
a) the particle loaded fibrous web exhibits in Cartesian coordinates
length direction, generally aligned with MD;
width direction, generally aligned with CD, and
thickness direction, generally aligned with the z-direction
each of the continuous operating machine; and
comprises
an aggregation of fibers,
a plurality of particles
positioned in interfiber interstices of the web or on the x-y-extending surfaces of the web,
b) the apparatus comprises a first and a further web guide,
the first web guide being a guide roll
adapted to move around an axis essentially aligned in CD of the machine, the guide roll further exhibiting
a radial coordinate r, aligned with the z-direction of the web when the web is in tangential positioning to the guide roll, and
an angular coordinate Φ, aligned with the x-direction of the web when the web is in a tangential positioning to the guide roll,
wherein the orientation of both the +x- and the +Φ -direction correspond to the orientation of the MD-direction upon movement of the web during operation of the apparatus;
the guide roll further comprising
a guide roll surface;
a plurality of protrusions positioned on and extending r-directionally from the surface of the web guide roll; and
a plurality of particle displacer tools, wherein a particle displacer tool is positioned on and extending r-directionally from the surface of the web guide roll, and positioned +Φ -directionally relative to a protrusion;
c) the web guides forming a gap adapted to receive said web
between the surface of the guide roll and the further web guide,
and exhibiting a gap width aligned with the z-direction of the web;
d) wherein a protrusion comprises
a protrusion base oriented towards the axis of the guide roll,
and a protrusion apex corresponding to the portion that extends r-directionally most outwardly away from the axis of the guide roll, and
a protrusion stem connecting said protrusion base and said protrusion apex;
e) wherein a particle displacer tool comprises
a displacer tool base oriented towards the axis of the guide roll,
a displacer tool apex corresponding to the portion that extends r-directionally most outwardly away from the axis of the guide roll, and
a leading ridge that extends from the displacer tool base to the displacer tool apex along the most forwardly positioned points of the displacer tool in the +Φ -direction at any radial position,
wherein a projection of the leading ridge on the Φ direction that is longer than the projection of the displacer tool on the cross-direction;
f) wherein the protrusion apex exhibits a flexibility in the -r direction of less than 30 µm, preferably less than 10 µm and more preferably less than 5 µm at a load of 200 N applied -r-directionally to the apex;
g) wherein further the apparatus comprises bonding elements selected from the group consisting of
g1) energy supply to enable melt fusion bonding, preferably selected from the group consisting of
i) heating;
ii) pressurizing;
iii) applying ultrasonic energy;
g2) adhesive supply, preferably to the apex of a protrusion.

2. An apparatus according to claim 1, wherein at least one of the conditions is satisfied that is selected from the group consisting of
a) a protrusion is essentially integral with the guide roll;
b) a particle displacer tool is essentially integral with the guide roll;
c) a particle displacer tool is essentially integral with a protrusion.

3. An apparatus according to any of the preceding claims, wherein said plurality of protrusions is forming a protrusion pattern, wherein a straight line connecting neighboring protrusions forms preferably an angle of more than 0°, more preferably more than 5° or even more preferably more than 10° to the Φ - direction of the guide roll.

4. A process for creating particle free regions in a particle loaded fibrous web on an apparatus for continuous operation according to any of the preceding claims,
the process comprising the steps of
- providing
a) a particle loaded fibrous web
comprising an aggregation of fibers and a plurality of particles positioned in interfiber interstices of the web or on the x-y-extending surfaces of the web,
b) a first and a further web guide,
the first web guide being a guide roll exhibiting a radial coordinate r, an angular coordinate Φ, aligned with the machine direction of the apparatus when the web is in a tangential positioning to the guide roll, and a cross-direction perpendicular to the machine direction;
the guide roll further comprising
a guide roll surface, a plurality of protrusions positioned on and extending r-directionally from the surface of the web guide roll, and a plurality of particle displacer tools positioned on and extending r-directionally from the surface of the web guide roll;
c) the web guides forming a nip
between the surface of the guide roll and the further web guide,
adapted to receive the web and
exhibiting a nip width;
- feeding said particle loaded web into said nip,
whilst concurrently rotating said guide roll such that said displacer tools penetrate into said web prior to said protrusions.
thereby dislocating particles cross-directionally and creating a particle-free region corresponding to the apex of the displacer tool when the apex is in its point of culmination relative to the further web,
wherein said apex of said protrusion dislocates less than 30 µm, preferably less than 20 µm and even more preferably less than 10 µm at a load of 200 N applied in the -r direction.

5. A process according to claim 4, wherein said particle loaded fibrous web comprises thermoplastic material,
said process further comprising the step of creating meltfusion bonding in said particle free region, by applying energy selected from the group consisting of pressure, heat, sonic, and ultrasonic energy.

6. A process according to claim 4, further comprising the step of applying a further material to said particle free regions, wherein preferably said further material is a treatment fluid.

7. A process according to claim 6, wherein said further material is a treatment fluid, and wherein said treatment fluid is a liquid bonding agent, preferably hot melt adhesive.

8. A process according to claim 7, wherein said particle loaded fibrous web comprises non-thermoplastic material.

9. A process according to any of the preceding claims, wherein said particle loaded fibrous web comprises sub-webs.

10. A process for forming a liquid absorbent structure, said process comprises steps according to any of claims 4 to 9, wherein said particle loaded fibrous web is liquid absorbing and said particles are preferably superabsorbent polymer particles.

11. A process for forming absorbent articles, comprising the step of forming a liquid absorbent structure according to claim 10.

## Patentansprüche

1. Kontinuierlich arbeitende Vorrichtung zum Erzeugen partikelfreier Bereiche in einer partikelbeladenen Faserbahn,
wobei die Vorrichtung in kartesischen Koordinaten
- eine Maschinenrichtung (MD), die den Weg der Faserstoffbahn definiert,
- eine Quermaschinenrichtung (CD) senkrecht dazu und
- eine z-Richtung senkrecht sowohl zu MD als auch zu CD
Aufzeigt, worin
a) die partikelbeladene Faserstoffbahn in kartesischen Koordinaten
- eine Längsrichtung, welche im Wesentlichen an MD der Vorrichtung ausgerichtet ist;
- eine Breitenrichtung, welche im Wesentlichen an CD der Vorrichtung ausgerichtet ist, und eine Dickenrichtung, welche im Wesentlichen an die z-Richtung der Vorrichtung ausgerichtet ist, aufweist,
und welche eine Ansammlung von Fasern und eine Vielzahl von Partikeln, die in Faserzwischenräumen der Bahn oder auf den x-y-erstreckenden Oberflächen der Bahn positioniert sind, umfasst;
b) die Vorrichtung eine erste und eine weitere Bahnführung umfasst,
wobei die erste Bahnführung eine Führungsrolle ist, welche angepasst ist sich um eine Achse zu bewegen, die im Wesentlichen in CD der Maschine ausgerichtet ist,
wobei die Führungsrolle weiterhin eine radiale Koordinate r aufweist, die an der z-Richtung der Bahn ausgerichtet ist, wenn die Bahn tangential zur Führungsrolle positioniert ist, und eine Winkelkoordinate Φ aufweist, die an der x-Richtung der Bahn ausgerichtet ist, wenn die Bahn tangential positioniert,
wobei die Orientierung sowohl der +x- als auch der +Φ-Richtung der Orientierung der MD-Richtung bei der Bewegung der Bahn während des Betriebs der Vorrichtung entspricht;
wobei die Führungsrolle ferner umfasst:
eine Führungsrollenoberfläche;
eine Vielzahl von Erhebungen, die auf der Oberfläche der Führungsrolle positioniert sind und sich in r-Richtung von dieser erstrecken; und
eine Vielzahl von Partikelverdrängerwerkzeugen, wobei ein Partikelverdrängerwerkzeug auf der Oberfläche der Führungsrolle positioniert ist und sich von dieser in r-Richtung erstreckt und in +Φ-Richtung relativ zu einer Erhebung positioniert ist;
c) wobei die Bahnführungen einen Spalt bilden, der geeignet ist, die Bahn aufzunehmen zwischen der Oberfläche der Führungsrolle und der weiteren Bahnführung, und eine Spaltbreiteaufweist, die mit der z-Richtung der Bahn ausgerichtet ist;
d) wobei eine Erhebung umfasst:
- eine Erhebungsbasis, die zur Achse der Führungsrolle ausgerichtet ist,
- und einen Erhebungsscheitel, der dem Abschnitt entspricht, der sich in r-Richtung am weitesten nach außen von der Achse der Führungsrolle weg erstreckt, und
- einen Erhebungsschaft, der die Erhebungsbasis und den Erhebungsscheitel verbindet;
e) wobei ein Partikelverdrängerwerkzeug umfasst:
- eine Verdrängerwerkzeugbasis, die zur Achse der Führungsrolle ausgerichtet ist, einen Verdrängerwerkzeugscheitel, die dem Abschnitt entspricht, der sich in r-Richtung am weitesten nach außen von der Achse der Führungsrolle weg erstreckt, und
- einen vorderen Grat, der sich von der Basis des Verdrängerwerkzeugs zum Scheitel des Verdrängerwerkzeugs entlang der am weitesten vorne positionierten Punkte des Verdrängerwerkzeugs in der +Φ-Richtung an jeder radialen Position erstreckt,
wobei eine Projektion des Führungsgrats in der Φ-Richtung länger ist als die Projektion des Verdrängerwerkzeugs in der Querrichtung;
f) wobei der Erhebungsscheitel eine Flexibilität in -r-Richtung von weniger als 30 µm, vorzugsweise weniger als 10 µm und besonders bevorzugt weniger als 5 µm bei einer Last von 200 N aufweist, die auf den Scheitel in -r-Richtung aufgebracht wird;
g) wobei die Vorrichtung ferner Verbindungselemente umfasst, ausgewählt aus der Gruppe bestehend aus
g1) Energiezufuhr zur Ermöglichung des Schmelzverbindens, vorzugsweise ausgewählt aus der Gruppe bestehend aus
i) Erhitzen;
ii) unter Druck setzen;
iii) Anwenden von Ultraschallenergie;
g2) Klebstoffzufuhr, vorzugsweise auf den Scheitel einer Erhebung.

2. Vorrichtung nach Anspruch 1, wobei mindestens eine der Bedingungen erfüllt ist, die ausgewählt ist aus der Gruppe bestehend aus
a) eine Erhebung ist im Wesentlichen einstückig mit der Führungsrolle;
b) ein Partikelverdrängerwerkzeug ist im Wesentlichen einstückig mit der Führungsrolle;
c) ein Partikelverdrängerwerkzeug ist im Wesentlichen einstückig mit einer Erhebung.

3. Vorrichtung nach einem der vorhergehenden Ansprüche, wobei die Vielzahl von Erhebungen ein Erhebungsmuster bildet, wobei eine gerade Linie, die benachbarte Erhebungen verbindet, vorzugsweise einen Winkel von mehr als 0°, bevorzugterweise mehr als 5° oder noch bevorzugterweise mehr als 10° zur Φ - Richtung der Führungsrolle bildet.

4. Ein Verfahren zum Erzeugen partikelfreier Bereiche in einer partikelbeladenen Faserbahn an einer kontinuierlich arbeitenden Vorrichtung gemäß eines der vorstehenden Ansprüche, wobei das Verfahren die Schritte umfasst:
- Bereitstellung
a) einer partikelbeladenen Faserbahn
umfassend eine Ansammlung von Fasern und einer Vielzahl von Partikeln, die in Faserzwischenräumen des Vlieses oder auf den sich x-y-erstreckenden Oberflächen der Faserbahn positioniert sind,
b) einer ersten und einer weiteren Bahnführung,
wobei die erste Bahnführung eine Führungsrolle ist, die eine radiale Koordinate r, eine Winkelkoordinate Φ aufweist, die mit der Maschinenrichtung der Vorrichtung ausgerichtet ist, wenn die Bahn tangential zur Führungsrolle positioniert ist, und eine Querrichtung senkrecht zur Maschinenrichtung;
wobei die Führungsrolle ferner umfasst:
eine Führungsrollenoberfläche, eine Vielzahl von Erhebungen, die auf der Oberfläche der Führungsrolle positioniert sind und sich in r-Richtung von der Oberfläche der Führungsrolle erstrecken, und eine Vielzahl von Partikelverdrängerwerkzeugen, die auf der Oberfläche der Bahnführungsrolle positioniert sind und sich in r-Richtung von dieser erstrecken;
c) wobei die Bahnführungen einen Walzenspalt bilden
zwischen der Oberfläche der Führungsrolle und der weiteren Bahnführung, angepasst, um das Web aufzunehmen und
eine Spaltbreite aufweisend;
- Zuführen der partikelbeladenen Bahn in den Walzenspalt, während gleichzeitig die Führungsrolle gedreht wird, so dass die Verdrängerwerkzeuge vor den Erhebungen in die Bahn eindringen.
wodurch Partikel in Querrichtung verschoben werden und ein partikelfreier Bereich entsprechend dem Scheitel des Verdrängerwerkzeugs erzeugt wird, wenn sich der Scheitel in seinem Kulminationspunkt relativ zur weiteren Bahn befindet,
wobei sich der Scheitel der Erhebung um weniger als 30 µm, vorzugsweise weniger als 20 µm und noch bevorzugterweise weniger als 10 µm in der -r-Richtung verlagert.

5. Verfahren nach Anspruch 4, wobei die partikelbeladene Faserbahn thermoplastisches Material umfasst,
wobei das Verfahren ferner den Schritt des Erzeugens einer Schmelzverbindung in dem partikelfreien Bereich durch Anwenden von Energie umfasst, die aus der Gruppe bestehend aus Druck, Wärme, Schall und Ultraschallenergie ausgewählt wird.

6. Verfahren nach Anspruch 4, ferner umfassend den Schritt des Aufbringens eines weiteren Materials auf die partikelfreien Bereiche, wobei das weitere Material vorzugsweise ein Behandlungsfluid ist.

7. Verfahren nach Anspruch 6, wobei das weitere Material ein Behandlungsfluid ist und wobei das Behandlungsfluid ein flüssiges Bindemittel ist, vorzugsweise ein Schmelzklebstoff.

8. Verfahren nach Anspruch 7, wobei die partikelbeladene Faserbahn ein nicht thermoplastisches Material umfasst.

9. Verfahren nach einem der vorhergehenden Ansprüche, wobei die partikelbeladene Faserbahn Unterbahnen umfasst.

10. Verfahren zum Bilden einer flüssigkeitsabsorbierenden Struktur, wobei das Verfahren Schritte nach einem der Ansprüche 4 bis 9 umfasst, wobei die partikelbeladene Faserbahn flüssigkeitsabsorbierend ist und die Partikel vorzugsweise superabsorbierende Polymerpartikel sind.

11. Verfahren zum Bilden absorbierender Artikel, umfassend den Schritt des Bildens einer flüssigkeitsabsorbierenden Struktur nach Anspruch 10.

## Revendications

1. Appareil à fonctionnement continu pour créer des régions exemptes de particules dans une nappe fibreuse chargée de particules,
ledit appareil présentant en coordonnées cartésiennes
- un sens machine (MD) définissant le chemin de la nappe fibreuse,
- un sens travers (CD) perpendiculaire à celui-ci et
- une direction z perpendiculaire à la fois à MD et à CD ;
où
a) la nappe fibreuse chargée de particules présente en coordonnées cartésiennes
- sens de la longueur, généralement aligné avec MD;
- sens de la largeur, généralement aligné avec le CD, et
- direction de l'épaisseur, généralement alignée avec la direction z
chacun par rapport au appareil à fonctionnement continu;
et comprend
une agrégation de fibres et une pluralité de particules positionné dans les interstices interfibres de la nappe ou sur les surfaces s'étendant x-y de la nappe ;
b) l'appareil comprend un premier et un autre guide de bande,
le premier guide de bande étant un rouleau de guidage adapté pour se déplacer autour d'un axe essentiellement aligné dans le CD de la machine,
le rouleau de guidage présentant en outre
- une coordonnée radiale r, alignée avec la direction z de la bande lorsque la bande est dans un positionnement tangentiel au rouleau de guidage, et
une coordonnée angulaire, alignée avec la direction x de la bande lorsque la bande est dans un positionnement tangent à le rouleau de guidage,
dans lequel l'orientation à la fois de la direction +x et de la direction +Φ correspond à l'orientation de la direction MD lors du mouvement de la bande pendant le fonctionnement de l'appareil ;
le rouleau de guidage comprenant en outre
une surface de rouleau de guidage ;
une pluralité de saillies positionnées sur et s'étendant dans la direction r depuis la surface du rouleau de guidage ; et une pluralité d'outils de déplacement de particules,
dans lequel un outil de déplacement de particules est positionné sur et s'étendant dans la direction r depuis la surface du rouleau de guidage, et positionné dans une direction +Φ - par rapport à une saillie ;
c) les guides de bande formant une fente adaptée pour recevoir ladite bande entre la surface du rouleau de guidage et l'autre guide de bande, et présentant une largeur fente alignée avec la direction z de la bande ;
d) dans lequel une saillie comprend
- une base de saillie orientée vers l'axe du rouleau de guidage,
- et un sommet de saillie correspondant à la partie qui s'étend dans la direction r le plus vers l'extérieur en s'éloignant de l'axe du rouleau de guidage, et
- une tige de saillie reliant ladite base de saillie et ledit sommet de saillie ;
e) dans lequel un outil de déplacement de particules comprend
- une base d'outil de déplacement orientée vers l'axe du rouleau de guidage,
- un sommet d'outil de déplacement correspondant à la partie qui s'étend dans la direction r le plus vers l'extérieur à l'écart de l'axe du rouleau de guidage, et
- une arête antérieure qui s'étend depuis la base de l'outil de déplacement jusqu'au sommet de l'outil de déplacement le long des points positionnés les plus en avant de l'outil de déplacement dans la direction +Φ à n'importe quelle position radiale,
dans lequel une projection de l'arête antérieure sur la direction Φ qui est plus longue que la projection de l'outil de déplacement sur la direction transversale ;
f) dans laquelle le sommet de la saillie présente une flexibilité dans la direction -r inférieure à 30 µm, de préférence inférieure à 10 µm et plus préférablement inférieure à 5 µm à une charge de 200 N appliquée dans le sens -r-directionnel au sommet ;
g) dans lequel l'appareil comprenant en outre des éléments de liaison choisis dans le groupe constitué par
g1) alimentation d'énergie pour permettre la liaison par fusion, de préférence choisie dans le groupe constitué par
i) chauffage ;
ii) la mise sous pression ;
iii) application de l'énergie ultrasonore ;
g2) alimentation d'un adhésif, de préférence au sommet d'une saillie.

2. Appareil selon la revendication 1, dans lequel au moins l'une des conditions est satisfaite qui est choisie dans le groupe constitué de
a) une saillie est essentiellement solidaire du rouleau de guidage ;
b) un outil de déplacement de particules est essentiellement solidaire du rouleau de guidage ;
c) un outil de déplacement de particules est essentiellement intégré à une saillie.

3. Appareil selon l'une quelconque des revendications précédentes, dans lequel ladite pluralité de saillies forme un motif de saillies, dans lequel une ligne droite reliant des saillies voisines forme de préférence un angle supérieur à 0°, plus préférablement supérieur à 5° ou encore plus préférablement supérieur à 10° à la direction Φ - du rouleau de guidage.

4. Procédé de création de régions exemptes de particules dans une nappe fibreuse chargée de particules sur un appareil à fonctionnement continu selon l'une quelconque des revendications,
le procédé comprenant les étapes de
- fournir
a) une nappe fibreuse chargée de particules
comprenant un agrégat de fibres et une pluralité de particules positionnées dans des interstices interfibres de la nappe ou sur les surfaces s'étendant x-y de la nappe,
b) un premier et un autre guide de nappe,
- le premier guide de bande étant un rouleau de guidage présentant une coordonnée radiale r, une coordonnée angulaire Φ, alignée avec la direction machine de l'appareil lorsque la bande est dans un positionnement tangentiel au rouleau de guidage, et une direction transversale perpendiculaire à la direction de la machine;
- le rouleau de guidage comprenant en outre
- une surface de rouleau de guidage,
- une pluralité de saillies positionnées sur et s'étendant dans la direction r depuis la surface du rouleau de guidage et
- une pluralité d'outils de déplacement de particules positionnés sur et s'étendant dans la direction r depuis la surface du rouleau de guidage;
c) les guides de bande formant une fente entre la surface du rouleau de guidage et l'autre guide de bande, adapté pour recevoir la nappe et
présentant une largeur fente;
- alimenter ladite bande chargée de particules dans ladite fente, tout en faisant tourner simultanément ledit rouleau de guidage de telle sorte que lesdits outils de déplacement pénètrent dans ladite bande avant lesdites saillies.
- disloquant ainsi les particules de manière transversale et créant une région sans particules correspondant au sommet de l'outil de déplacement lorsque le sommet est à son point culminant par rapport à l'autre bande,
dans lequel ledit sommet de ladite saillie se disloque à moins de 30 µm, de préférence à moins de 20 µm et encore plus préférablement à moins de 10 µm dans la direction -r.

5. Procédé selon la revendication 4, dans lequel ladite nappe fibreuse chargée de particules comprend un matériau thermoplastique,
ledit procédé comprenant en outre l'étape de création d'une liaison par fusion dans ladite région exempte de particules, en appliquant une énergie choisis dans le groupe constitué par pression, chaleur, énergie sonique et ultrasonore.

6. Procédé selon la revendication 4, comprenant en outre l'étape d'application d'un autre matériau auxdites régions exemptes de particules, dans lequel de préférence ledit autre matériau est un fluide de traitement.

7. Procédé selon la revendication 6, dans lequel ledit autre matériau est un fluide de traitement, et dans lequel ledit fluide de traitement est un agent de liaison liquide, de préférence un adhésif thermofusible.

8. Procédé selon la revendication 7, dans lequel ladite nappe fibreuse chargée de particules comprend un matériau non thermoplastique.

9. Procédé selon l'une quelconque des revendications précédentes, dans lequel ladite nappe fibreuse chargée de particules comprend des sous- nappes.

10. Procédé de formation d'une structure absorbant les liquides, ledit procédé comprenant des étapes selon l'une quelconque des revendications 4 à 9, dans lequel ladite nappe fibreuse chargée de particules absorbe les liquides et lesdites particules sont de préférence des particules de polymère superabsorbant.

11. Procédé de formation d'articles absorbants, comprenant l'étape de formation d'une structure absorbant les liquides selon la revendication 10.
